# EUROPÄISCHE PATENTSCHRIFT

(11) **EP 0 767 469 B1**
(45) Veröffentlichungstag und Bekanntmachung des Hinweises auf die Patenterteilung: **17.07.2002**
(21) Anmeldenummer: 96402029.1
(22) Anmeldetag: 25.09.1996
(51) Int. Cl.: H01B 9/00, H01B 7/04

(54) **Flexible Leitung**
Flexible line
Ligne flexible

(30) Priorität: 02.10.1995 DE 29515705 U
(43) Veröffentlichungstag der Anmeldung: 09.04.1997
(73) Patentinhaber: Nexans, 75008 Paris (FR)
(72) Erfinder: Warden, Gert, 41199 Mönchengladbach (DE)
(74) Vertreter: Döring, Roger, Dipl.-Ing.

(56) Entgegenhaltungen:
- DE-A- 2 818 492
- DE-U- 8 628 059

## Beschreibung

Die Erfindung betrifft eine flexible Leitung zur Energieübertragung mit einer zentralen Energieader, einer Mehrzahl von über der zentralen Energieader verseilten äußeren Energieadern, deren Isolierung aus einem gummmielastischen Werkstoff besteht, und einem Außenmantel aus einem gummielastischen Werkstoff, der alle Energieadern umschließt (DE 86 28 059 U).

Derartige flexible Leitungen werden beispielsweise auf Flughäfen als Bordspeiseleitungen zur Energieübertragung und gegebenenfalls zur Übertragung von Steuersignalen bei an einem Flugsteig angedockten Flugzeugen verwendet. Da solche Bordspeiseleitungen im Betrieb hohen mechanischen Beanspruchungen, wie Auf- und Abtrommeln, Entlangschleifen auf dem Boden sowie Verdrehungen, ausgesetzt sind, weisen sie einen diesen Belastungen gewachsenen Aufbau des Außenmantels auf. Um den Anforderungen an eine hohe Flexibilität der Leitung gerecht zu werden, bestehen die Isolierungen der Energieadern sowie der Außenmantel der Leitung aus gummielastischen Werkstoffen. Bekannte flexible Leitungen haben den Nachteil, daß sie nach einem Biegevorgang, also beispielsweise nach dem Abtrommeln, kein Bestreben haben, sich in ihre langgestreckte Form zu legen.

Aus dem eingangs erwähnten DE 86 28 059 U geht ein flexibles mehradriges Kabel hervor. Dieses Kabel weist miteinander verseilte Energieadern auf. Die Energieadern haben Litzenleiter und sind gegeneinander Isoliert. Über den Aufbau der Leiter und deren Isolierung sind keine Angaben gemacht. In den äußeren Zwickeln der Energieadern sind Steueradern angeordnet, die erheblich dünner als die Energieadern sind. Zu ihrem Schutz sind die mechanisch nur gering beanspruchbaren Steueradern in Schutzrohren untergebracht.

Der Erfindung liegt die Aufgabe zugrunde, die eingangs geschilderte Leitung so weiterzubilden, daß ihre Elastizität erhöht und ihre Rückstellkräfte vergrößert werden.

Diese Aufgabe wird gemäß der Erfindung dadurch gelöst,
- daß die zentrale Energieader einen aus einer Vielzahl von miteinander verseilten Leiterdrähten gebildeten elektrischen Leiter hat, dessen Leiterdrähte mit einer einem Steigungswinkel von kleiner als 25° entsprechenden kurzen Schlaglänge miteinander verseilt sind und,
- daß die über dem Leiter aufgebrachte Isolierung der zentralen Energieader aus einem Werkstoff mit einem Elastizitätsmodul von zumindest 500 N/mm² besteht.

Diese flexible Leitung weist eine große Elastizität und hohe Rückstellkräfte auf, die bestrebt sind, die Leitung nach einem Biegevorgang wieder in ihre langgestreckte, gerade Form zu bringen. Das wird durch die kurze Schlaglänge der verseilten Leiterdrähte einerseits sowie durch die aus einem Werkstoff mit hohem Elastizitätsmodul bestehende Isolierung der zentralen Energieader andererseits erreicht. Die kurze Schlaglänge der Leiterdrähte verhindert darüber hinaus die Gefahr von Brüchen der einzelnen Leiterdrähte. Durch den Elastizitätsmodul von zumindest 500 N/mm² für den Werkstoff der Isolierung der zentralen Energieader ist die Isolierung vergleichsweise steif und hart, so daß sie nach einem Biegevorgang der Leitung eine entsprechend große Rückstellkraft auf dieselbe ausübt.

Die trotz ihres großen Rückstellbestrebens sehr flexible Leitung kann beispielsweise als Bordspeiseleitung zur Energieübertragung für an einem Flugsteig angedockte Flugzeuge, aber auch als Aufzugsleitung, trommelbare Leitung oder Schleppkettenleitung verwendet werden.

In den Unteransprüchen sind vorteilhafte Weiterbildungen und Verbesserungen der Erfindung beschrieben.

Um eine möglichst große Rückstellwirkung der zentralen Energieader und damit der flexiblen Leitung insgesamt zu erreichen und die Gefahr von Brüchen der Leiterdrähte zuverlässig zu vermeiden, ist es von Vorteil, wenn der zwischen den verseilten Leiterdrähten und dem Querschnitt des elektrischer, Leiters der zentralen Energieader gebildete Steigungswinkel kleiner als 15° ist.

Für einen wirksamen Schutz der Energieadern vor unzulässig hohen Zugbeanspruchungen ist es von Vorteil, wenn jede der Energieadern von einer Folie umschlossen ist, über der ein zugentlastendes Geflecht angeordnet ist.

Darüber hinaus ist es von Vorteil, wenn alle Energieadern von einem gemeinsamen Innenmantel umschlossen sind, über dem ein Außenmantel aufgebracht ist, der eine innere Schicht, in die ein zugentlastendes Geflecht eingebettet ist, und eine äußere Schicht aufweist, so daß die Leitung gut vor unzulässig hohen Zugbeanspruchungen geschützt ist.

Um über die Leitung auch Steuersignale übertragen zu können, ist es vorteilhaft, wenn in zwischen den verseilten äußeren Energieadern gebildeten Zwickeln Steueradern angeordnet sind.

Ein Ausführungsbeispiel des Erfindungsgegenstandes ist in der Zeichnung dargestellt.

Es zeigen:
- Fig. 1: einen Querschnitt einer erfindungsgemäßen flexiblen Leitung.
- Fig. 2: eine abgesetzte zentrale Energieader der Leitung nach Fig. 1.

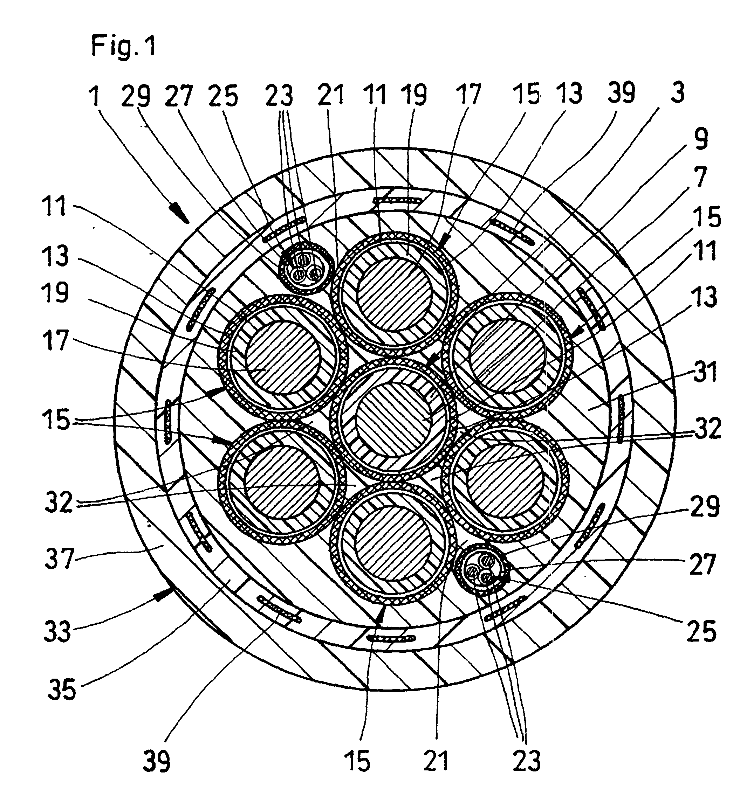

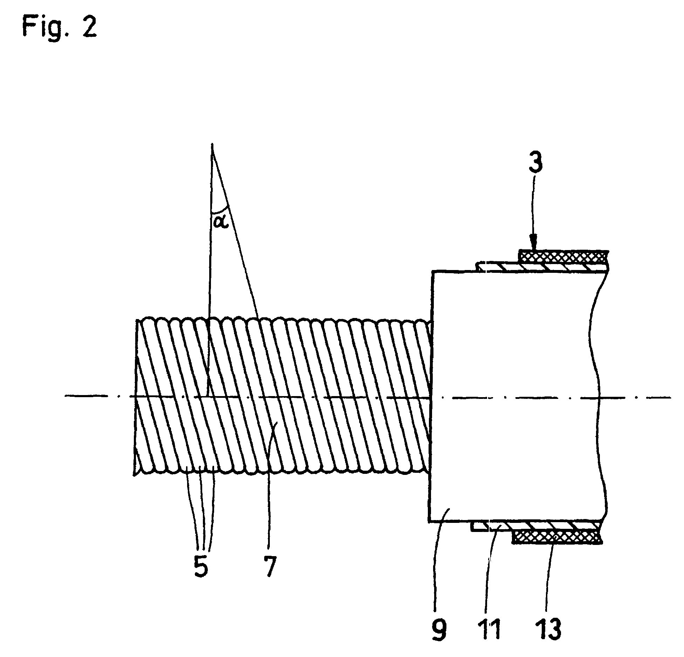

Die in Fig. 1 beispielhaft dargestellte flexible Leitung 1, die sich z. B. als Bordspeiseleitung zur Energieübertragung und zur Übertragung von Steuersignalen bei an einem Flugsteig angedockten Flugzeugen eignet, weist eine zentrale Energieader 3 auf, die in Fig. 2 genauer dargestellt ist. Die zentrale Energieader 3 hat einen aus einer Vielzahl von mit kurzer Schlaglänge verseilten Leiterdrähten 5 gebildeten elektrischen Leiter 7. Der Steigungswinkel α (Fig. 2) der Leiterdrähte 5 ist als Maßgabe für die kurze Schlaglänge kleiner als 25° und beispielsweise kleiner als 15°.

Über dem Leiter 7 der zentralen Energieader 3 ist eine Isolierung 9 aus einem Kunststoff mit hohem Elastizitätsmodul aufgebracht. Ein derartiger Kunststoff wird von Fachleuten auch als "hart" bezeichnet. Dieser Werkstoff weist einen Elastizitätsmodul von zumindest 500 N/mm² auf, der wesentlich höher als der Elastizitätsmodul von üblicherweise verwendeten gummielastischen Werkstoffen ist. Als Werkstoff für die Isolierung 9 ist z. B. entweder Polyester oder Polypropylen verwendet. Die Isolierung 9 der zentralen Energieader 3 kann von einer Folie 11 umschlossen sein, über der ein zugentlastendes Geflecht 13 aus hochzugfesten Elementen angeordnet ist.

Über der zentralen Energieader 3 sind beispielsweise sechs äußere Energieadern 15 verseilt, die jeweils einen elektrischen Leiter 17 und eine darüber liegende Isolierung 19 aus einem gummielastischen Werkstoff aufweisen. Jede der äußeren Energieadern 15 ist von einer Folie 11 umschlossen, über der ein zugentlastendes Geflecht 13 aus hochzugfesten Elementen, wie z. B. Fäden, angeordnet ist.

Zur Übertragung von Steuersignalen ist z. B. in zwei zwischen den verseilten äußeren Energieadern 15 gebildeten äußeren Zwickeln 21 jeweils ein beispielsweise aus drei miteinander verseilten, elektrisch isolierten Steueradern 23 bestehender Steueraderverband 25 angeordnet. Die beiden Steueraderverbände 25 sind jeweils von einer Folie 27 umschlossen, über der ein zugentlastendes Geflecht 29 aus hochzugfesten Elementen angeordnet ist.

Die miteinander verseilten äußeren Energieadern 15 und die in den äußeren Zwickeln 21 verlaufenden Steueraderverbände 25 sind von einem aus einem gummielastischen Werkstoff bestehenden Innenmantel 31 umschlossen, der die äußeren Zwickel 21 und z. B. auch die zwischen der zentralen Energieader 3 und den darüber verseilten äußeren Energieadern 15 gebildeten inneren Zwickel 32 ausfüllt. Über dem Innenmantel 31 ist ein Außenmantel 33 aus einem gummielastischen Werkstoff aufgebracht, der beispielsweise aus einer inneren Schicht 35 und einer verschleißfesten äußeren Schicht 37 besteht. In die innere Schicht 35 des Außenmantels 33 ist beispielsweise ein zugentlastendes Geflecht 39 aus hochzugfesten Elementen, wie z. B. Fäden, eingebettet. Es ist aber ebenfalls möglich, anstelle des zugfesten Geflechtes 39 einzelne zugfeste Elemente zu verwenden.

## Patentansprüche

1. Flexible Leitung zur Energieübertragung mit einer zentralen Energieader, einer Mehrzahl von über der zentralen Energieader verseilten äußeren Energieadern, deren Isolierung aus einem gummielastischen Werkstoff besteht, und einem Außenmantel aus einem gummielastischen Werkstoff, der alle Energieadern umschließt, **dadurch gekennzeichnet,**
- **daß** die zentrale Energieader (3) einen aus einer Vielzahl von miteinander verseilten Leiterdrähten (5) gebildeten elektrischen Leiter (7) hat, dessen Leiterdrähte (5) mit einer einem Steigungswinkel (α) von kleiner als 25° entsprechenden kurzen Schlaglänge miteinander verseilt sind, und
- **daß** die über dem Leiter (7) aufgebrachte Isolierung (9) der zentralen Energieader (3) aus einem Werkstoff mit einem Elastizitätsmodul von zumindest 500 N/mm² besteht.

2. Leitung nach Anspruch 1, **dadurch gekennzeichnet, daß** der Steigungswinkel (α) der Leiterdrähte (5) kleiner als 15° ist.

3. Leitung nach Anspruch 1 oder 2, **dadurch gekennzeichnet, daß** die Isolierung (9) der zentralen Energieader (3) aus Polyester besteht.

4. Leitung nach Anspruch 1 oder 2, **dadurch gekennzeichnet, daß** die Isolierung (9) der zentralen Energieader (3) aus Polypropylen besteht.

5. Leitung nach einem der Ansprüche 1 bis 4, **dadurch gekennzeichnet, daß** jede der Energieadern (3,15) von einer Folie (11) umschlossen ist, über der ein zugentlastendes Geflecht (13) angeordnet ist.

6. Leitung nach einem der Ansprüche 1 bis 5, **dadurch gekennzeichnet, daß** die Energieadern (3,15) von einem gemeinsamen Innenmantel (31) umschlossen sind, über welchem der Außenmantel (33) angebracht ist.

7. Leitung nach einem der Ansprüche 1 bis 6, **dadurch gekennzeichnet, daß** der Außenmantel (33) eine innere Schicht (35), in die ein zugentlastendes Geflecht (39) eingebettet ist, und eine äußere Schicht (37) aufweist.

8. Leitung nach einem der Ansprüche 1 bis 7, **dadurch gekennzeichnet, daß** in zwischen den verseilten äußeren Energieadern (15) gebildeten Zwickeln (21) Steueradern (23) angeordnet sind.

## Claims

1. A flexible line for energy transfer with a central energy cable, a plurality of outer stranded energy cables that are provided over the central energy cable, the insulation of which outer energy cables consists of an elastic rubber material, and an outer shell of elastic rubber material that encompasses all energy cables, **characterized in that**
- the central energy cable (3) comprises an electric conductor (7) formed by means of a plurality of stranded conductor wires (5), which conductor wires (5) are stranded with each other with a short length of lay that corresponds to a pitch gradient (α) of less than 25°, and
- that the insulation (9) of the central energy cable (3) that is provided over the conductor (7) consists of a material having an elastic modulus of at least 500 N/mm².

2. The line according to claim1, **characterized in that** the pitch gradient (α) of the conductor wires (5) is less than 15°.

3. The line according to claims 1 or 2, **characterized in that** the insulation (9) of the central energy cable (3) consists of polyester.

4. The line according to claims 1 or 2, **characterized in that** the insulation (9) of the central energy cable (3) consists of polypropylene.

5. The line according to one of claims 1 through 4, **characterized in that** each of the energy cables (3, 15) are enveloped by a film (11) over which a strain relief braided sleeving (13) is disposed.

6. The line according to one of claims 1 through 5, **characterized in that** the energy cables (3, 15) are enveloped by a common inner shell (31), over which the outer shell (33) is disposed.

7. The line according to one of claims 1 through 6, **characterized in that** the outer shell (33) exhibits an inner layer (35), in which a strain relief braided sleeving (39) is embedded, and an outer layer (37).

8. The line according to one of claims 1 through 7, **characterized in that** pilot wires (23) are disposed in the gaps (21) that are formed between the outer stranded energy cables (15).

## Revendications

1. Câble flexible pour la transmission d'énergie avec un brin central de transmission d'énergie, un nombre multiple de brins extérieurs de transmission d'énergie toronnés par-dessus le brin central, dont l'isolation est composée d'un matériau élastique comme du caoutchouc, et dune gaine extérieure en matériau élastique comme du caoutchouc enrobant tous les conducteurs, **caractérisée en ce que**
- le brin central de transmission d'énergie (3) possède un conducteur électrique (7) formé par un grand nombre de fils conducteurs (5) toronnés entre eux, dont les fils conducteurs (5) sont toronnés entre eux par un pas de câblage court correspondant à un angle d'hélice (α) inférieur à 25° et **en ce que**
- l'isolation (9) appliquée sur le conducteur (7) du brin central de transmission d'énergie (3) se compose d'un matériau avec un module d'élasticité d'au moins 500 N/mm².

2. Conduite selon la revendication 1, **caractérisée en ce que** l'angle d'hélice (α) des fils conducteurs (5) est inférieur à 15°.

3. Conduite selon la revendication 1 ou 2, **caractérisée en ce que** l'isolation (9) du brin central de transmission d'énergie (3) est en polyester.

4. Conduite selon la revendication 1 ou 2, **caractérisée en ce que** l'isolation (9) du brin central de transmission d'énergie (3) est en polypropylène.

5. Conduite selon l'une des revendications 1 à 4, **caractérisée en ce que** chaque brin de transmission d'énergie (3, 15) est entouré d'un film (11) par-dessus duquel est disposé une tresse (13) réduisant la traction.

6. Conduite selon l'une des revendications 1 à 5, **caractérisée en ce que** les brins de transmission d'énergie (3, 15) sont entourés par une gaine intérieure (31) commune par-dessus de laquelle est disposée la gaine extérieure (33).

7. Conduite selon l'une des revendications 1 à 6, **caractérisée en ce que** la gaine extérieure (33) présente une couche intérieure (35), dans laquelle est disposé une tresse (39) réduisant la traction, et une couche extérieure (37).

8. Conduite selon l'une des revendications 1 à 7, **caractérisée en ce que** des brins pilotes (23) sont disposés dans les bourrages (21) formés entre les brins de transmission d'énergie extérieurs toronnés (15).
